# EUROPÄISCHE PATENTSCHRIFT

(11) **EP 0 359 956 B1**
(45) Veröffentlichungstag und Bekanntmachung des Hinweises auf die Patenterteilung: **13.07.1994**
(21) Anmeldenummer: 89114314.1
(22) Anmeldetag: 03.08.1989
(51) Int. Cl.: C07C 213/04, C07C 215/06, B01D 3/12

(54) **Verfahren und Vorrichtung zur Herstellung von 1,3-Diaminopropanol-2**
Process and apparatus for the preparation of 1,3-diamino-2-propanol
Procédé et appareil de préparation de 1,3-diamino-2-propanol

(30) Priorität: 23.09.1988 DE 3832372
(43) Veröffentlichungstag der Anmeldung: 28.03.1990
(73) Patentinhaber: DEUTSCHE SOLVAY-WERKE GMBH, 42697 Solingen (DE)
(72) Erfinder: Jakobson, Gerald, Dr., D-4134 Rheinberg 2 (DE); Klumpe, Michael, Dr., D-4134 Rheinberg 1 (DE)
(74) Vertreter: Lauer, Dieter, Dr.

(56) Entgegenhaltungen:
- DE-A- 1 941 859
- US-A- 3 432 553
- US-A- 4 795 565
- PATENT ABSTRACTS OF JAPAN, Band 4, Nr. 7 (C-70), 19. Januar 1980, Seite 47 C 70; & JP-A-54 141 709

## Beschreibung

Die vorliegende Erfindung betrifft ein Verfahren und eine Vorrichtung zur Herstellung von 1,3-Diaminopropanol-2 durch Umsetzung von Epichlorhydrin mit wäßriger Ammoniaklösung, die Ammoniak im Überschuß bezogen auf Epichlorhydrin enthält, wobei Epichlorhydrin in einem Überschuß an Ammoniak unter Einhaltung eines bestimmten Molverhältnisses eingesetzt und umgesetzt und das gebildete Reaktionsprodukt einem bestimmten Reinigungsverfahren zur Gewinnung von 1,3-Diaminopropanol-2 unterworfen wird.

Es ist bereits bekannt, eine Sauerstoffgruppe enthaltende aliphatische Verbindung im Überschuß mit Ammoniak zur Herstellung eines Gemisches von Alkanolaminen umzusetzen (vgl. DE-OS 1 941 859). Bei diesem Verfahren muß unter sehr hohen Drucken gearbeitet werden. Dabei gelingt es nur ein Gemisch von Aminen oder Alkanolaminen herzustellen, insbesondere nicht das 1,3-Diaminopropanol-2 mit einer entsprechenden Ausbeute. Darüber hinaus wird nicht von Epichlorhydrin ausgegangen.

In der US-A-3,432,553 wird ein zweistufiges Verfahren zur Herstellung von 1,3-Diaminopropanol-2 aus Epichlorhydrin und einer wäßrigen Ammoniaklösung beschrieben. Es handelt sich dabei um ein Verfahren, daß wegen seiner mehrstufigen Reaktionsführung, seinem hohen Überschuß an Ammoniak (bezogen auf das eingesetzte Epichlorhydrin) und der aufwendigen Abtrennung gebildeter anorganischer Salze während der Umsetzung hohe Kosten bei der Herstellung von 1,3-Diaminopropanol-2 verursacht.

Aufgabe der vorliegenden Erfindung war es, ein verbessertes Verfahren zur Herstellung von 1,3-Diaminopropanol-2 ausgehend von Epichlorhydrin durch Umsetzung von im Überschuß angewendetem Ammoniak zu finden. Das Verfahren sollte konstante Ausbeuten an reinem 1,3-Diaminopropanol-2 liefern. Weiterhin sollte innerhalb des Verfahrens die Filtration von Salzen vermieden werden.

Erfindungsgemäß wurde festgestellt, daß diesen Zielen und Aufgaben ein Verfahren zur Herstellung von 1,3-Diaminopropanol-2 durch Umsetzung von Epichlorhydrin mit wäßriger Ammoniaklösung gerecht wird, bei dem Ammoniak im Überschuß bezogen auf Epichlorhydrin eingesetzt wird. Gemäß der Erfindung werden pro Mol Epichlorhydrin 15 bis 24 Mole Ammoniak eingesetzt. Das gebildete Reaktionsprodukt wird während oder nach der Reaktion mit einer stark oder sehr stark basischen Anionenaustauschermasse in Kontakt gebracht, nachfolgend Ammoniak und Wasser unter vermindertem Druck abgedampft und aus dem Reaktionsprodukt 1,3-Diaminopropanol-2 durch Destillation gewonnen. Nach einer vorzugsweisen Ausführungsform werden pro Mol Epichlorhydrin 16 bis 23 Mol Ammoniak eingesetzt.

Das abgedampfte Ammoniak wird dabei zur Umsetzung von Epichlorhydrin zurückgeführt und im Kreislauf geführt.

Die stark basische Anionenaustauschermasse wird bevorzugt in einem molaren oder größer als molaren Verhältnis, bezogen auf die eingesetzte Menge Epichlorhydrin, eingesetzt.

Nach einer vorzugsweisen Ausführungsform des erfindungsgemäßen Verfahren wird die nach der Umsetzung von Ammoniak mit Epichlorhydrin gebildete Reaktionslösung über mindestens einen stark basischen Anionenaustauscher bei Temperaturen unter 30 °C geleitet und unmittelbar oder unter Zwischenschaltung von mindestens einem Vorratsbehälter zu mindestens einer Verdampfungsvorrichtung, vorzugsweise mindestens einem Fallfilm- oder Kurzwegverdampfer, überführt und dort bei Temperaturen über 50 °C unter Unterdruck Ammoniak und Wasser abgedampft.

In der Verdampfungsvorrichtung wird in Abhängigkeit von dem vorhandenen Ammoniak und der Temperatur ein Unterdruck von kleiner als 500 mbar eingehalten und Ammoniak und Wasser bei Temperaturen über 50 °C abgedampft; vorzugsweise wird hierbei ein Unterdruck von kleiner als 400 mbar eingehalten. Nach einer bevorzugten Ausführungsform wird das abgedampfte Ammoniak vor der Umsetzung mit Epichlorhydrin auf die Anwendungskonzentration (größer 10 %, vorzugsweise größer 20 %) mit Wasser oder Ammoniaklösung eingestellt.

Die nach der Entfernung des Wassers und Ammoniak erhaltene Reaktionslösung, vorzugsweise die mehr als 50 °C warme Reaktionslösung wird bei Temperaturen von
80 bis 160 °C
und einem Druck von
1 bis 25 mbar
einer Vordestillation unterworfen; vorzugsweise wird die Vordestillation bei einer Temperatur von 90 bis 130 °C und einem Druck von 2 bis 10 mbar ausgeführt.

Das nach der Vordestillation erhaltene Destillat wird einer Feindestillation am Kurzwegverdampfer bei Temperaturen über 90 °C bei einem Druck von 5 bis 15 mbar unterworfen. Vorzugsweise wird die Feindestillation bei 100 bis 110 °C bei einem Druck von 10 bis 12 mbar ausgeführt.

Als wäßrige Ammoniaklösung wird eine Ammoniaklösung in einer Konzentration von mehr als 10 Gew.-% Ammoniak, vorzugsweise mehr als 20 Gew.-% Ammoniak, eingesetzt. Nach einer vorzugsweisen Ausführungsform wird eine wäßrige Ammoniaklösung mit einem Ammoniakgehalt von 21 Gew.-% bis 40 Gew.-% verwendet.

Die zur Umsetzung verwendete wäßrige Ammoniaklösung enthält nach der vorzugsweisen Ausführungsform keine Alkali- oder Erdalkalihydroxide oder diese nur in Gewichtsmengen unter 2 Gew.-%, vorzugsweise unter 1 Gew.-%. Bei der Reaktion von Epichlorhydrin mit Ammoniak wird die Reaktionslösung vorzugsweise gekühlt und die Temperatur so gesteuert, daß die Temperatur der Reaktionslösung unter ca. 35 °C, vorzugsweise unter 30 °C, gehalten wird.

Die wäßrige ammoniakhaltige Reaktionslösung wird nach der Umsetzung durch mindestens einen stark basischen Anionenaustauscher bei einem Druck von mindestens 1 bar, vorzugsweise 1,001 bis 3 bar, geleitet. Dabei werden vorzugsweise Pumpen oder andere Druckregulierungsmittel eingesetzt.

Nach dem erfindungsgemäßen Verfahren werden bevorzugt Epichlorhydrin und Ammoniak in Abwesenheit zusätzlicher organisch-chemischer wasserunlöslicher Lösemittel oder nur mit einem organisch-chemischen wasserunlöslichen Lösemittelgehalt von weniger als 5 Gew.-%, vorzugsweise weniger als 2 Gew.-% (bezogen auf die Gesamtmenge an Epichlor-hydrin und Ammoniak), umgesetzt; hierbei wird die Reaktionstemperatur auf 1 bis 30 °C, vorzugsweise 3 bis 27 °C, eingestellt. Im Rahmen des erfindungsgemäßen Verfahrens können bei der Umsetzung von Epichlorhydrin mit Ammoniak auch organisch-chemische wasserlösliche Lösemittel, z.B. Methanol, Ethanol, Aceton und dgl. mitverwendet werden, so daß das Wasser bis zu 50 Gew.-% (bezogen auf 100 Gew.-% Verdünnungsmittel, d.h. Wasser und Lösemittel) durch dieses wasserlösliche Lösemittel ersetzt werden kann. Bevorzugt wird Wasser als alleiniges Lösemittel eingesetzt.

Das erfindungsgemäße Verfahren hat die Vorteile, daß konstante Ausbeuten an reinem 1,3-Diaminopropanol-2 erzielt werden können. Während der Reaktionsführung ist eine Abfiltration gebildeter anorganischer Salze nicht notwendig. Organische wasserunlösliche Lösemittel können innerhalb des Verfahrens vermieden werden. Das Verfahren ist umweltfreundlich, da das nicht verbrauchte Ammoniak im Kreislauf geführt wird. Die Anwendung von Überdruck ist bei der Reaktion nicht erforderlich.

Die Erfindung betrifft weiterhin eine Vorrichtung zur Herstellung von 1,3-Diaminopropanol-2 aus Epichlorhydrin und Ammoniaklösung, bestehend aus mindestens einem Reaktionsgefäß, mindestens einer Verdampfungs- oder Destillationsvorrichtung sowie ggf. einem Vorratsbehälter, wobei das Reaktionsgefäß mit mindestens einer Anionenaustauschvorrichtung in Verbindung steht, die unmittelbar oder über einen Vorratsbehälter mit mindestens einer Verdampfungsvorrichtung verbunden ist, von der mindestens eine Verdampfungsvorrichtung eine Fallfilmverdampfungsvorrichtung ist, die mit einer Vorrichtung zur Erzielung eines Vakuums kombiniert ist.

Nach einer bevorzugten Ausführungsform ist nach der Fallfilmverdampfungsvorrichtung mindestens eine Kurzwegverdampfervorrichtung angeordnet.

### Ausführungsbeispiele:

### Beispiel 1

2,5 l wässrige NH₃-Lösung (25 %ig, entsprechend 36,8 Mole Ammoniak) werden auf 5 - 10 °C abgekühlt und bei dieser Temperatur in 2 - 4 h mit 177 g (entsprechend 1,9 mol) Epichlorhydrin versetzt. Nach Beendigung der Zugabe wird die Lösung noch 1 h gerührt und dann über eine stark basische Anionenaustauschsäule (Lewatit ® MB 600 G 3; 2 - 2,5 l stark basische Anionenaustauschermasse, entsprechend 1,9 bis 2,3 Val Bindungskapazität) gepumpt. Der Austauscher wird nach dem Durchlauf der Reaktionslösung mit 2,5 l Wasser nachgewaschen und anschließend die Gesamtlösung in einer Verdampfungsvorrichtung unter vermindertem Druck (400 - 30 mbar) bei Temperaturen zwischen 50 und 60 °C von Ammoniak und Wasser befreit.

Der erhaltene Reaktionsrückstand wird dann destillativ vorgereinigt (Fraktionierung bei 95 - 115 °C; 2 - 10 mbar) und das erhaltene farblose Destillat anschließend am Kurzwegverdampfer gereinigt (102 - 104 °C; 10 mbar).

Man erhält 91 g 1,3-Diaminopropanol-2 (entsprechend 53 % d. Th.).

### Beispiel 2

20 l NH₃-Lösung (25 %ig) werden bei Raumtemperatur in 4 - 5 h mit 1,18 kg (entsprechend 12,7 mol) Epichlorhydrin entsprechend einem Molverhältnis 1 : 23,1 versetzt, wobei darauf geachtet wird, daß die Temperatur der Reaktionslösung nicht über 30 °C ansteigt. Die so erhaltene Lösung wird anschließend durch 15 - 17 l stark basische Anionenaustauschermasse (entsprechend 13,5 - 15,3 Val Bindungskapazität) bei einer Temperatur unterhalb 30 °C gepumpt und der Austauscher mit 10 - 12 l Wasser nachgespült. Die Gesamtlösung wird dann am Kurzwegverdampfer von NH₃ und H₂O befreit (90 - 95 °C; 200 - 40 mbar). Das abgedampfte NH₃ wird dabei in vorgekühltes Wasser eingeleitet und die so erhaltene NH₃-Lösung wieder zur Darstellung von 1,3-Diaminopropanol-2 verwendet.

Das Restwasser wird anschließend am Rotationsverdampfer abgezogen und der erhaltene Rückstand wie in Beispiel 1 destilliert.

Man erhält 588 g 1,3-Diaminopropanol-2 (entsprechend 52 % d.Th.).

### Beispiel 3

10 l 25%ige NH₃-Lösung werden zusammen mit 10 l stark basischer Anionenaustauschermasse entsprechend 9 Val Bindungskapazität in einem 20 l-Reaktor gebracht und die Lösung auf 8 - 10 °C gekühlt. Unter Kühlung werden innerhalb von 2 - 3 h 590 g (= 6,3 mol) Epichlorhydrin (Molverhältnis 1 : 23,3) zugetropft und die Lösung nach Ende der Zugabe nach 1 h weiter gekühlt. Anschließend wird ohne Kühlung noch 3 - 4 h gerührt und die Lösung dann vom Ionenaustauscher getrennt. Die Austauschermasse wird mit 5 - 6 l Wasser nachgewaschen und die Gesamtlösung am Rotationsverdampfer bei 65 °C, 300 - 30 mbar von Ammoniak und Wasser befreit.

Der Rückstand wird wie in Beispiel 1 vordestilliert und dann am Kurzwegverdampfer feindestilliert.

Man erhält 316 g (entsprechend 55% d.Th.) 1,3-Diaminopropanol-2.

## Patentansprüche

1. Verfahren zur Herstellung von 1,3-Diaminopropanol-2 durch Umsetzung von Epichlorhydrin mit wäßriger Ammoniaklösung, die Ammoniak im Überschuß bezogen auf Epichlorhydrin enthält, dadurch gekennzeichnet, daß pro Mol Epichlorhydrin 15 bis 24 Mole Ammoniak eingesetzt werden, daß das gebildete Reaktionsprodukt während oder nach der Reaktion mit einer stark basischen Anionenaustauschermasse in Kontakt gebracht, nachfolgend Ammoniak und Wasser unter vermindertem Druck abgedampft und aus dem Reaktionsprodukt 1,3-Diaminopropanol-2 durch Destillation gewonnen wird.

2. Verfahren nach Anspruch 1, dadurch gekennzeichnet, daß das abgedampfte Ammoniak zur Umsetzung von Epichlorhydrin zurückgeführt und im Kreislauf geführt wird.

3. Verfahren nach Ansprüchen 1 und 2, dadurch gekennzeichnet, daß die stark basische Anionenaustauschermasse in einem molaren oder größer als molaren Verhältnis, bezogen auf die eingesetzte Menge Epichlorhydrin, eingesetzt wird.

4. Verfahren nach einem oder mehreren der Ansprüche 1 bis 3, dadurch gekennzeichnet, daß die nach Umsetzung von Ammoniak mit Epichlorhydrin gebildete Reaktionslösung über mindestens einen stark basischen Anionenaustauscher bei Temperaturen unter 30 °C geleitet und unmittelbar oder unter Zwischenschaltung von mindestens einem Vorratsbehälter zu mindestens einer Verdampfungsvorrichtung, vorzugsweise mindestens einem Fallfilm- oder Kurzwegverdampfer, überführt und dort bei Temperaturen über 50 °C unter Unterdruck Ammoniak und Wasser abgedampft wird.

5. Verfahren nach einem oder mehreren der Ansprüche 1 bis 4, dadurch gekennzeichnet, daß in der Verdampfungsvorrichtung in Abhängigkeit von dem vorhandenen Ammoniak und der Temperatur ein Unterdruck von
kleiner als 500 mbar,
eingehalten und Ammoniak und Wasser bei Temperaturen über 50 °C abgedampft werden.

6. Verfahren nach einem oder mehreren der Ansprüche 1 bis 5, dadurch gekennzeichnet, daß die nach Entfernung des Wassers und Ammoniak erhaltene Reaktionslösung, vorzugsweise die mehr als 50 °C warme Reaktionslösung, bei Temperaturen von
80 bis 160 °C,
und einem Druck von
1 bis 25 mbar,
einer Vordestillation unterworfen wird.

7. Verfahren nach einem oder mehreren der Ansprüche 1 bis 6, dadurch gekennzeichnet, daß das nach der Vordestillation erhaltene Destillat einer Feindestillation am Kurzwegverdampfer bei Temperaturen über 90 °C, und bei einem Druck von
5 bis 15 mbar
unterworfen wird.

8. Verfahren nach einem oder mehreren der Ansprüche 1 bis 7, dadurch gekennzeichnet, daß als wäßrige Ammoniaklösung eine Ammoniaklösung in einer Konzentration von
mehr als 10 Gew.-% Ammoniak,
eingesetzt wird.

9. Verfahren nach einem oder mehreren der Ansprüche 1 bis 8, dadurch gekennzeichnet, daß die zur Umsetzung verwendete wäßrige Ammoniaklösung keine Alkali- oder Erdalkalihydroxide oder diese nur in Gewichtsmengen
unter 2 Gew.-%,
enthält.

10. Verfahren nach einem oder mehreren der Ansprüche 1 bis 9, dadurch gekennzeichnet, daß die wäßrige und ammoniakhaltige Reaktionslösung durch mindestens einen stark basischen Anionenaustauscher bei einem Druck von mindestens 1 bar geleitet wird.

11. Verfahren nach einem oder mehreren der Ansprüche 1 bis 10, dadurch gekennzeichnet, daß Epichlorhydrin und Ammoniak in wäßriger Lösung in Abwesenheit zusätzlicher organisch-chemischer wasserunlöslicher Lösemittel oder nur mit einem organisch-chemischen wasserunlöslichen Lösemittelgehalt von
weniger als 5 Gew.-%,
(bezogen auf die Gesamtmenge an Epichlorhydrin und Ammoniak), umgesetzt werden, wobei die Reaktionstemperatur auf
1 bis 30 °C,
eingestellt wird.

12. Vorrichtung zur Herstellung von 1,3-Diaminopropanol-2 aus Epichlorhydrin und Ammoniaklösung, bestehend aus mindestens einem Reaktionsgefäß, mindestens einer Verdampfungs- oder Destillationsvorrichtung sowie ggf. einem Vorratsbehälter, dadurch gekennzeichnet, daß das Reaktionsgefäß mit mindestens einer Anionenaustauschvorrichtung in Verbindung steht, die unmittelbar oder über einen Vorratsbehälter mit mindestens einer Verdampfungsvorrichtung verbunden ist, von der mindestens eine Verdampfungsvorrichtung eine Fallfilmverdampfungsvorrichtung ist, die mit einer Vorrichtung zur Erzielung eines Vakuums kombiniert ist.

13. Vorrichtung nach Anspruch 12, dadurch gekennzeichnet, daß nach der Fallfilmverdampfungsvorrichtung mindestens eine Kurzwegverdampfervorrichtung angeordnet ist.

## Claims

1. A method for the preparation of 1,3-diaminopropanol-2 by reacting epichlorohydrin with aqueous ammonia solution which contains ammonia in an excess relative to the epichlorohydrin, characterised in that 15 to 24 moles ammonia are used per mole epichlorohydrin, that the resulting reaction product during or after the reaction is brought into contact with a strongly basic anion-exchanger substance, subsequently ammonia and water are evaporated off under reduced pressure and 1,3-diaminopropanol-2 is obtained from the reaction product by distillation.

2. A method according to Claim 1, characterised in that the ammonia evaporated off is returned for the reaction of epichlorohydrin and is recycled.

3. A method according to Claims 1 and 2, characterised in that the strongly basic anion-exchanger substance is used in a molar or greater than molar ratio, relative to the quantity of epichlorohydrin used.

4. A method according to one or more of Claims 1 to 3, characterised in that the reaction solution formed after the reaction of ammonia with epichlorohydrin is passed over at least one strongly basic anion exchanger at temperatures below 30°C and is transferred, directly or with the interposition of at least one storage vessel, to at least one evaporation device, preferably at least one falling-film or short-path evaporator, and ammonia and water are evaporated off therein at temperatures of above 50°C under reduced pressure.

5. A method according to one or more of Claims 1 to 4, characterised in that a reduced pressure of
less than 500 mbar
is maintained in the evaporation device dependent on the ammonia present and the temperature, and ammonia and water are evaporated off at temperatures above 50°C.

6. A method according to one or more of Claims 1 to 5, characterised in that the reaction solution obtained after the removal of the water and ammonia, preferably the reaction solution which is at a temperature of more than 50°C, is subjected to preliminary distillation at temperatures of
80 to 160°C
and a pressure of
1 to 25 mbar.

7. A method according to one or more of Claims 1 to 6, characterised in that the distillate obtained after the preliminary distillation is subjected to fine distillation on the short-path evaporator at temperatures of above 90°C, and at a pressure of
5 to 15 mbar.

8. A method according to one or more of Claims 1 to 7, characterised in that an ammonia solution in a concentration of
more than 10% by weight ammonia
is used as the aqueous ammonia solution.

9. A method according to one or more of Claims 1 to 8, characterised in that the aqueous ammonia solution used for the reaction contains no alkali or alkaline earth hydroxides or only contains these in quantities of
less than 2% by weight.

10. A method according to one or more of Claims 1 to 9, characterised in that the aqueous and ammonia-containing reaction solution is passed through at least one strongly basic anion exchanger at a pressure of at least 1 bar.

11. A method according to one or more of Claims 1 to 10, characterised in that epichlorohydrin and ammonia in aqueous solution in the absence of additional organic-chemical, water-insoluble solvent or only with a content of organic-chemical, water-insoluble solvent of
less than 5% by weight
(relative to the total quantity of epichlorohydrin and ammonia) are reacted, the reaction temperature being set to
1 to 30°C.

12. An apparatus for the preparation of 1,3-diaminopropanol-2 from epichlorohydrin and ammonia solution, consisting of at least one reaction vessel, at least one evaporation or distillation apparatus and optionally a storage vessel, characterised in that the reaction vessel communicates with at least one anion-exchange apparatus which communicates directly or by means of a storage vessel with at least one evaporation apparatus, of which at least one evaporation apparatus is a falling-film evaporation apparatus, which is combined with an apparatus for obtaining a vacuum.

13. An apparatus according to Claim 12, characterised in that at least one short-path evaporation apparatus is arranged after the falling-film evaporation apparatus.

## Revendications

1. Procédé de préparation de 1,3-diaminopropanol-2 par la réaction de l'épichlorhydrine avec une solution aqueuse d'ammoniac (ou ammoniaque) qui contient de l'ammoniac en excès par rapport à l'épichlorhydrine, procédé caractérisé en ce que, par mole d'épichlorhydrine, on utilise 15 à 24 moles d'ammoniac, en ce qu'on met, pendant ou après la réaction, le produit formé par cette réaction en contact avec une masse fortement basique échangeuse d'anions, puis on chasse par évaporation sous pression réduite l'ammoniac et l'eau et l'on récupère par distillation du produit de la réaction le 1,3-diaminopropanol-2.

2. Procédé selon la revendication 1, caractérisé en ce qu'on recycle, pour le faire réagir avec l'épichlorhydrine, l'ammoniac chassé par évaporation et on le fait circuler en circuit fermé.

3. Procédé selon les revendications 1 et 2, caractérisé en ce qu'on utilise la masse échangeuse d'anions, fortement basique, en un rapport équimolaire ou supérieur au rapport équimolaire, par rapport à la quantité d'épichlorhydrine utilisée.

4. Procédé selon une ou plusieurs des revendications 1 à 3, caractérisé en ce qu'on achemine sur au moins un échangeur d'anions fortement basique, à des températures inférieures à 30°C, la solution de réaction formée après la réaction de l'ammoniac avec l'épichlorhydrine et l'on achemine cette solution, immédiatement ou après interposition d'au moins un réservoir, vers au moins un dispositif d'évaporation, avantageusement au moins un évaporateur à film tombant ou à court trajet de distillation, et l'on en chasse, par évaporation à des températures supérieures à 50°C et sous dépression, l'amonniac et l'eau.

5. Procédé selon une ou plusieurs des revendications 1 à 4, caractérisé en ce que dans l'appareil d'évaporation, on maintient, en fonction de l'ammoniac présent et de la température, une dépression inférieure à 500 mbar, et l'on chasse par évaporation l'ammoniac et l'eau à des températures supérieures à 50°C.

6. Procédé selon une ou plusieurs des revendications 1 à 5, caractérisé en ce qu'on soumet la solution de réaction, obtenue après l'élimination de l'eau et de l'ammoniac, avantageusement la solution de réaction chaude, qui est à une température supérieure à 50°C, à une prédistillation effectuée à des températures de 80 à 160°C et sous une pression de 1 à 25 mbars.

7. Procédé selon une ou plusieurs des revendications 1 à 6, caractérisé en ce qu'on soumet le distillat, obtenu après la prédistillation, à une distillation fine sur un évaporateur à court trajet à des températures supérieures à 90°C et sous une pression de 5 à 15 mbars.

8. Procédé selon une ou plusieurs des revendications 1 à 7, caractérisé en ce qu'on utilise comme solution aqueuse d'ammoniac ou comme ammoniaque une solution d'ammoniac ayant une concentration supérieure à 10 % en poids d'ammoniac.

9. Procédé selon une ou plusieurs des revendications 1 à 8, caractérisé en ce que la solution aqueuse d'ammoniac utilisée pour la réaction ne contient pas d'hydroxydes alcalins ou alcalino terreux ou ne les contient qu'en des quantités pondérales inférieures à 2 %.

10. Procédé selon une ou plusieurs des revendications 1 à 9, caractérisé en ce qu'on achemine la solution aqueuse de réaction, contenant de l'ammoniac, en la faisant passer par au moins un échangeur fortement basique d'anions, sous une pression d'au moins 1 bar.

11. Procédé selon une ou plusieurs des revendications 1 à 10, caractérisé en ce qu'on fait réagir l'épichlorhydrine et l'ammoniac en solution aqueuse, en l'absence d'un solvant organique chimique additionnel, insoluble dans l'eau ou en présence d'une proportion de solvant organique chimique insoluble dans l'eau inférieure à 5 % en poids (par rapport à la quantité totale de l'épichlorhydrine et de l'ammoniac), en ajustant la température de réaction à 1 jusqu'à 30°C.

12. Appareil pour préparer le 1,3-diaminopropanol-2 à partir de l'épichlorhydrine et d'une solution d'ammoniac, l'appareil consistant en au moins un réacteur, au moins un dispositif d'évaporation ou de distillation et éventuellement un réservoir, appareil caractérisé en ce que le réacteur est en liaison avec au moins un dispositif échangeur d'anions qui est relié, immédiatement ou par l'intermédiaire d'un réservoir, à au moins un dispositif d'évaporation, dont au moins un est un dispositif d'évaporation à film tombant, qui est associé à un dispositif pour obtenir une dépression.

13. Appareil selon la revendication 12, caractérisé en ce qu'après le dispositif d'évaporation à film tombant, il y a au moins un dispositif d'évaporation à court trajet d'évaporation.
